# EUROPEAN PATENT APPLICATION

(11) **EP 3 228 240 A1**
(43) Date of publication of application: **11.10.2017**
(21) Application number: 16164516.3
(22) Date of filing: 08.04.2016
(51) Int. Cl.: A61B 5/00, A45D 2/00, A61B 5/04, G01N 27/60

(54) **FIBER QUALITY SENSOR**

(71) Applicant: Koninklijke Philips N.V., 5656 AE Eindhoven (NL)
(72) Inventor: Gerhardt, Lutz, 5656 AE Eindhoven (NL); Asvadi, Sima, 5656 AE Eindhoven (NL); van Lierop, Michiel, 5656 AE Eindhoven (NL); Joye, Neil, 5656 AE Eindhoven (NL); Johnson, Mark, 5656 AE Eindhoven (NL); Ouwerkerk, Martin, 5656 AE Eindhoven (NL)
(74) Representative: Steenbeek, Leonardus Johannes

(57) **Abstract**

In a fiber quality sensor (S) comprising a first pair of electrodes, the first pair of electrodes include a first conductive electrode (1), and a first dielectric electrode (D, 2) having a first dielectric material (D) with a conductive backing (2). The fiber quality sensor (S) may further comprise a second pair of electrodes, the second pair of electrodes including a second conductive electrode (1), and a second dielectric electrode (D, 2) having a second dielectric material with a conductive backing, wherein in a tribo-electric series of materials, the material of the conductive electrode is arranged between the first and second dielectric materials. The fiber quality sensor (S) may comprise horizontally or vertically alternating first and second pairs of electrodes. The first and/or second pairs of electrodes may be tilted, slanted and/or zigzag-shaped. The fiber quality sensor (S) may for the or each pair of electrodes further comprise an amplifier coupled to the pair of electrodes and having an input impedance exceeding 1 TΩ, and preferably being 200 TΩ. The fiber quality sensor (S) is advantageously applied in a hair care device.

## Description

### FIELD OF THE INVENTION

The invention relates to a fiber quality sensor such as a hair damage sensor, and to a hair care device comprising such a hair damage sensor.

### BACKGROUND OF THE INVENTION

The chapter 7 "Surface Potential Studies of Human Hair Using Kelvin Probe Microscopy", by Bhushan in: Biophysics of Human Hair: Structural, Nanomechanical, and Nanotribological Studies, pp. 153-169 (2010), discloses that virgin hair has a better charge mobility and can therefore dissipate charge more readily than chemically damaged hair.

### SUMMARY OF THE INVENTION

It is, inter alia, an object of the invention to provide a fiber quality sensor. The invention is defined by the independent claims. Advantageous embodiments are defined in the dependent claims.

Embodiments of the invention provide a hair based triboelectric sensor device with self-generating sensor signal to detect hair health or damage, and in particular a sensor device that measures and analyses the signal shape, magnitude of hair surface potential and rate of hair surface discharging (charge retention/dissipation) during a hair treatment with a hair care device, such as a straightener. The sensor technology is based on the analysis of electrostatic and/or triboelectric surface charging characteristics of hair fibers. The hair based sensing technology with self-generating signal offers potential for both a stand-alone consumer and/or professional device and an integrated module into existing hair styling devices (e.g. hair straightener, styler). The hair based sensor is based on but not limited to a sliding triboelectric generator being used as a sensor for measuring a charge (voltage or surface potential change) which is dependent on the surface property (topography, chemistry) of hair. The sensor principle makes use of the triboelectric effect, in-plane charge separation and electrostatic induction. The rubbing of a hair against a counter-surface (e.g. of a care device) causes an electrical charge build-up and charge induction in a specific sensor electrode array, made out of a conductive and dielectric electrode allows to monitor the time-dependent and spatial, macroscopic discharge behavior. The charge leaks away through the hair and/or sensor specific induction electrode materials/configurations. The rate of this process depends on the structural (integrity of cuticles, number of cuticle layers removed) and physicochemical surface properties of the hair fiber (presence of lipids, moisture level). By comparing the voltage signature with reference shapes and analyzing the signal in the time and frequency domain, the level of hair damaged can be assessed and quantified (if a calibration curve is available), or a baseline measurement done.

These and other aspects of the invention will be apparent from and elucidated with reference to the embodiments described hereinafter.

### BRIEF DESCRIPTION OF THE DRAWINGS

Fig. 1 illustrates the principle of the invention;
Fig. 2 shows a first embodiment of a hair damage sensor in accordance with the invention;
Fig. 3 shows a (multiple) interdigitated hybrid induction electrode pair embodiment for hair damage sensing/detection according to the invention;
Figs. 4A, 4B and 4C show different electrode arrangements on hair straightener plate to compensate for angular deviation and hair length difference at hair tips; and
Figs. 5, 6A and 6B show examples of electrode configurations for use in a fiber quality sensor according to the invention.

### DESCRIPTION OF EMBODIMENTS

Embodiments of the invention provide a hair based triboelectric sensor with self-generating sensor signal to detect hair health or damage. In particular, embodiments provide a sensor device that measures and analyses the signal shape, magnitude of hair surface potential and rate of hair surface discharging (charge retention/dissipation) during a hair treatment with a hair care device, such as a straightener. The sensor is based on a hair based sliding triboelectric generators for generating charge in response to movement of the hair care device over the hair; and this hair based triboelectric generator being used as a sensor for measuring a parameter which is dependent on the surface property (topography, chemistry), the sensor signal being the charge (voltage) generated by the triboelectric generator. The sensor principle makes use of the triboelectric effect, in-plane charge separation and electrostatic induction. The rubbing of a hair against a counter-surface (e.g. of a hair care device) causes an electrical charge build-up and charge induction in a specific sensor electrode array, made out of a conductive and dielectric electrode allows to monitor the time-dependent and spatial, macroscopic discharge behavior. The charge leaks away through the hair and/or sensor specific induction electrode materials/configurations. The rate of this process depends on the structural (integrity of cuticles, number of cuticle layers removed) and physicochemical surface properties of the hair fiber (presence of lipids, moisture level). By comparing the voltage signature with reference shapes and analyzing the signal in the time and frequency domain, the level of hair damaged can be assessed and quantified (if a calibration curve is available), or a baseline measurement done.

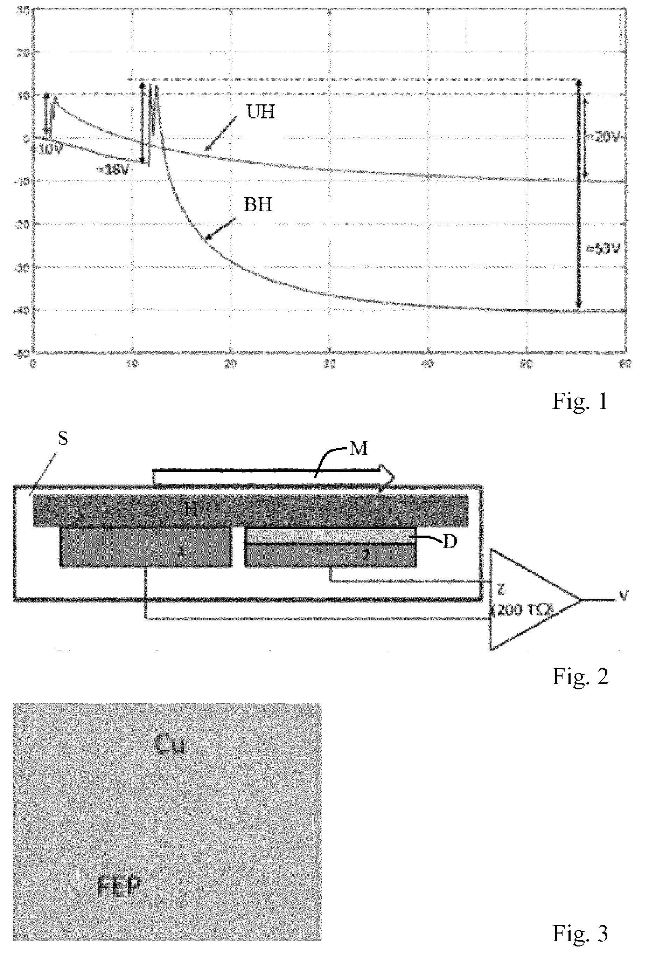

Fig. 1 shows triboelectric hair surface potential changes in Volt of untreated hair denoted by UH and bleached hair denoted by BH (using commercially available chemical products) versus time in seconds, measured during a one-directional stroke of blond hair over a specific electrode configuration subject of this invention. The curve BH shows that bleached hair can build up more surface charges (higher peak-peak voltage Vpp: 18 V vs. 10 V) but charges do leak away faster (no moisture repelling lipid layer) and to a greater extent (saturation voltage: -40 V vs -10 V) compared to the unbleached reference sample UH. The curve UH for unbleached hair shows an increase in potential (2^{nd} peak is larger than 1^{st} peak), whereas the charge leakage is faster for bleached hair BH indicated by the fact that the 2^{nd} peak is lower and/or as high as the 1^{st} peak. The minimum is also lower for bleached hair BH than for unbleached hair UH indicating greater leakage rate. Whereas the step amplitude ratios of the leading edge of two successive peaks are similar (12 V/18 V for bleached hair BH, 5 V / 8 V for unbleached hair UH) and presumably related to the (geometric) properties of the electrodes, the absolute peak height ratio of two successive peaks gives information on the leakage rate and charge retention properties of hair, and hair condition (e.g. presence of lipids, moisture level). Absolute peak height ratios <1 indicate charge leakage dominating charge build up in time (for bleached hair BH: 11.5 V / 12 V), ratios >1 indicate charge build up dominating charge leakage in time (for unbleached hair UH: 10 V / 8 V). It has to be noticed that the height of the 1^{st} and 2^{nd} peaks, and therefore the absolute peak height ratio, also depends on the triboelectric sensor configuration and speed of the stroke.

To be able to use this invention in a handheld device, an electrode pair or grating structure - together with the hair forming a sliding triboelectric generator - is integrated in the hair care device and responds to the surface charge build-up by means of triboelectric charging and electrostatic induction. Suitable electronics to capture the signal/charge build up is an amplifier with large input resistance (typically > 10¹² Ω).

In a first embodiment of this invention, a specific hybrid electrode design made out of conductive and dielectric electrodes allows to monitor the time-dependent and spatial, macroscopic discharge behavior. The conductive part is made of a conductive material such as copper (Cu), aluminum (Al) or a transparent conductor such as indium tin oxide (ITO). The dielectric part is made of a dielectric material such as polyester (PET) or fluorinated ethylene propylene (FEP). This invention is not limited to the listed materials. Many other possible materials could also be used. An in-plane induction electrode pair or multiple interdigitated electrodes made out of one metallized dielectric (metal is on the back side) and one conductive material enable to measure large flat hair bundles and assess the hair damage state based on controlled discharge through the conductive electrode(s). Hair rubbed over these electrode arrays result in multiple in-plane charge separation and induction events but also 'forced' discharge events due to conductive metal electrode allowing one to study the dynamic discharge behavior of hair.

Fig. 2 shows a first embodiment of a hair damage sensor in accordance with the present invention with alternating conductive and dielectric electrode segments. M indicates a unidirectional movement of the sensor S along the hair H. The sensor S includes two electrodes 1 and 2, the second electrode 2 being covered by a dielectric D. The output voltages of the electrodes 1 and 2 are applied to an amplifier having a very high input impedance Z of about 200 TΩ. The very large input impedance Z of the amplifier insures that the charges present on the electrodes 1, 2 leak very slowly into the amplifier. When being rubbed over such a dielectric-conductive electrode pair or alternating array of dielectric-conductive electrodes, a specific voltage potential across the electrode pair is generated. This voltage potential is due to the triboelectric charges and electrostatic induction being generated at the hair/sensor interface. Moreover, charge leakage from the hair to the conductive electrode 1 (e.g. copper electrode) has also an influence on the measured voltage. Thus, the voltage potential measured across the electrode pair (partially) depends on the hair surface topography/chemistry and therefore allows to characterize the health state of hair.

If the unique signal signature deviates, this allows a statement on the charging state/characteristics and surface condition of the hair (see embodiment 4).

All the herein described in-plane electrode pairs/arrays can, for example, be integrated in a plate of a hair straightener.

Fig. 3 shows a (multiple) interdigitated hybrid induction electrode pair embodiment for hair damage sensing/detection according to the invention. Dielectric = FEP; conductor = copper denoted by Cu. As in Fig. 1, the FEP electrode has a copper backing.

In a second embodiment of this invention, various geometries of hybrid electrodes are used to compensate for angular deviation and avoid signal noise at the hair tip during use of a hair care device. These geometries are aimed to improve the sensor signal. To fully exploit the sensor signal, in the ideal case the hair should be rubbed substantially perpendicularly relative to the orientation of the electrodes pairs. In many hair care applications it may be difficult to maintain or control such a substantially perpendicular orientation, or the device is used under a certain angle relative to the length of the hair bundle or root-tip direction. Therefore, specific electrode design configurations are needed making straightening robust to DC noise burst which would be generated instead of an alternating voltage signal during sliding at the hair tip. By using specific arrangements and modifications of the above described hybrid electrode (embodiment 1), such a noise burst can be avoided and clear signals extracted. For compensating angular deviation from a perfect 90 degree angle or angular misalignment, and improve the sensor signal (signal-to-noise ratio), dedicated geometries can include the following electrode geometries or combinations thereof:
- Slanted/tilted electrodes
- V-shaped electrodes
- zig-zag shaped electrodes
- Meander-like electrodes
- Staggered electrodes
- Alternating electrodes with asymmetric width of electrode fingers (e.g. width of conductive electrode twice the width of dielectric electrode to improve discharge events).

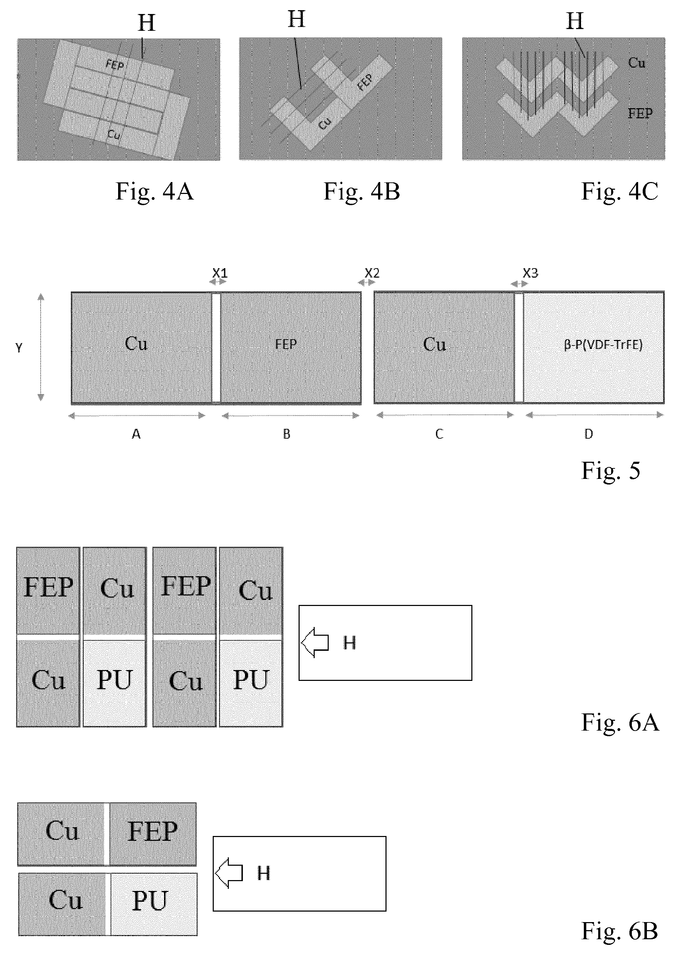

Figs. 4A, 4B and 4C show different electrode arrangements on hair straightener plate to compensate for angular deviation and hair length difference at hair tips. Fig. 4A shows slanted / tilted electrodes, Fig. 4B shows V-shaped electrodes, and Fig. 4C shows zig-zag shaped electrodes as can be provided on a straightener plate of a hair care device.

In a third embodiment, similar to embodiments 1 and 2, an in-plane electrode configuration of alternating electrodes materials is used. In this embodiment there is one additional material, and thus in total three types of materials, viz. (1) an electrically conductive material, such as a metal like copper (Cu), (2) a material which is lower than the chosen electrically conductive material in the triboelectric series compared to hair, for example fluorinated ethylene propylene (FEP) or Teflon types, and (3) a third material which is ranked above the chosen electrically conductive material (e.g. copper Cu) in the triboelectric series. Ideally, this third material should also be ranked above hair, e.g. polyurethane or negatively charged β-P(VDF-TrFE). See e.g. https://www.trifield.com/content/tribo-electric-series/, http://www.regentsprep.org/regents/physics/phys03/atribo/default.htm, or http://www.rfcafe.com/references/electrical/triboelectric-series.htm for a table listing various materials according to their respective affinities for negative charge.

The electrode configuration shown in Fig. 5 is used as an example. Similar to the previous embodiments, the voltage potential between electrodes A and B is monitored. Additionally, the voltage potential between electrodes C and D is also monitored. The configurations shown in Fig. 5 do not make the output "insensitive" to the brushing direction. To do this, one would need to "mirror" the configurations e.g. ABCDCDAB or even ABBACDDC. As in the previous embodiments, the FEP and β-P(VDF-TrFE) electrodes are metalized at the back. Symbols: A until D; X1 until X3 and Y denote the dimensions of the electrodes. These can be varied to get an optimal signal.

When hair strands are rubbed over the surface or vice versa, starting on the nonconductive segments and moving into the conductive segments, two electrical signals are generated. The usage of two triboelectric charge generating materials, one higher and one lower in the triboelectric series compared to copper, results in two electrical signals which are mainly in opposite direction of each other (respectively positively and negatively).

This additional signal and the ratio between these signals can be used to gain additional spatial information on the surface chemical state of hair (e.g. relative presence of polar and non-polar groups varying over the full hair length (from root to tip) or the individual cuticle length (typical 3-5 µm, i.e. suggested width of 3^{rd} electrode material is 1 to 3 µm). To compensate/nullify for/the misalignment (un-parallelism of active tribo areas in respect to cuticle direction), the electrode should have multiple small segments of e.g. 10 by 10 µm in length and/or width, preferably lengths and widths in the order of 1 to 5 µm. These dimensions of the active tribo area are not limited to the third material and can be used for one or more of the other used materials. The relatively uniform potential on the cuticle when measured far away from the cell borders increases close to the edge of the cuticle cells, indicating that these regions are more polar due to protein exposure.

Furthermore it can be used to distinguish between combing direction, from tip to root or from root to tip, as this difference in combing direction results in different electrical output signals.

By changing the electrode dimensions denoted in Fig. 5 by symbols A until D, X1-X3 and Y the electrical output signal can be adjusted. For example, the area of the FEP and β-P(VDF-TrFE) can be increased to increase the charging surface.

Furthermore multiple segments and combinations of these segments can used. For example, the configuration of Fig. 5 can be repeated horizontally and/or vertically. Figs. 6A and 6B show some other examples, with electrodes made of copper (Cu), fluorinated ethylene propylene (FEP) and polyurethane (PU), and these configurations too can be repeated horizontally and/or vertically. Fig. 6A shows alternating conductive and charge generating surfaces, and Fig. 6B shows both charge generating surfaces in front followed by the conductive segments in relation to the movement of the sensor over hair H.

In a fourth embodiment of this invention, the analysis of a modulated charge dissipating signal (which is dependent on the hair surface topography/chemistry properties and the electrode design such as width of electrode segments) allows to conclude on the hair damage state. As mentioned in relation to embodiment 2, the electrode layout dictates the shape of the generated triboelectric voltage. The signal is modulated and results in a distinctive pattern of repeated pulses. Damage detection of hair by signal analysis of distinctive triboelectric surface potential patterns is possible through:
- Comparison of triboelectric signal with deviation from standard signature of undamaged or untreated/unbleached hair.
- Characteristic shape of modulated signal in time domain or spatial domain: Relating time to displacement/movement during rubbing over electrode segments.
- Charge exchange, retention/dissipation rate (1^{st} derivative) of triboelectric surface potential signals: time derivative.
- Frequency spectra (harmonics, ratio of harmonics, frequency range).

Embodiments of the invention thus provide a new sensor technology with self-generating signal based on triboelectric effect and electrostatic charge induction. A specific electrode configuration is provided, with an array of two in-plane electrodes with alternating conductive and dielectric coated segments: alternating conductive (charge dissipation electrode) and dielectric covered induction electrodes. A main advantage of this feature is that it allows monitoring charge build up and discharge behavior/events of hair. This configuration fundamentally differs from a conventional triboelectric generator in that in such a conventional triboelectric generator a conductive discharge electrode would not be placed directly next to a dielectric electrode as this would negatively affect power generation/sensor signal magnitude as charges leak away. The controlled or forced sequential discharge monitoring of hair allows discriminating healthy from damaged hair based on charge leakage/dissipation behavior as shown in Fig. 1. We actually want to have such a controlled deliberate charge leakage in order to be able to characterize healthy and damaged hair based on typical discharge signals. Minimum additional electronics and system architectural changes are needed for implementation of sensor in hair straightener. A device with an in-built triboelectric sensor system for hear health sensing can be realized: no external power supply needed to power the sensor. Hair is part of a sensor system forming a triboelectric sensing device with self-generating signal. Aspects of the invention include:
- Sensor for sensing surface damage (e.g. coating imperfections/doped surfaces) based on triboelectric charges and electrostatic discharge phenomena.
- Hair based triboelectric sensor device with self-generating sensor signal to detect hair damage.
- Hair is part of a sensor system forming a triboelectric sensing device.
- Use triboelectric surface potential (tribo-voltages generated between the hair surface and the counter-surface of a hair care device) as an indicator/measure for hair health/damage.
- Methods to characterize macroscopic surface charging mechanisms of dielectric or badly conducting coatings: signal analysis in time and frequency domain.
- Methods to discriminate between undamaged and damaged hair: signal analysis in time and frequency domain.
- Handheld apparatus suitable for sensing surface damage (e.g. coating imperfections/doped surfaces) based on triboelectric charges and electrostatic discharge phenomena.
- Device inherent triboelectric sensor with self-generating sensor signal for monitoring hair health/damage.
- Handheld apparatus for triboelectric sensing of hair health and pre-warning for over-treatment.

The invention is advantageously used in a hair care device, comprising a fiber quality sensor S according to the invention, and a microprocessor coupled to receive output signals from the fiber quality sensor. The hair care device could, for example, provide an indication (e.g. optical or acoustic alarm) to the user that he/she should stop the treatment in order to avoid overheating. Settings (e.g. temperature) could also be adapted.

The invention can be applied not only in hair care applications, but also in textile (fiber) applications, e.g. monitoring quality and durability of textile finishing during production, or in garment care, e.g. monitoring quality and durability of textiles during use or after laundering.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. In the device claim enumerating several means, several of these means may be embodied by one and the same item of hardware. The mere fact that certain measures are recited in mutually different dependent claims does not indicate that a combination of these measures cannot be used to advantage.

## Claims

1. A fiber quality sensor (S), comprising a first pair of electrodes, the first pair of electrodes including:
a first conductive electrode (1, Cu), and
a first dielectric electrode (D, 2) having a first dielectric material (D, FEP) with a conductive backing (2).

2. A fiber quality sensor (S) as claimed in claim 1, the sensor further comprising a second pair of electrodes, the second pair of electrodes including
a second conductive electrode (1, Cu), and
a second dielectric electrode (D, 2) having a second dielectric material (PU) with a conductive backing, wherein in a tribo-electric series of materials the material of the conductive electrode (Cu) is arranged between the first (FEP) and second (PU) dielectric materials.

3. A fiber quality sensor (S) as claimed in claim 2, comprising horizontally or vertically alternating first and second pairs of electrodes.

4. A fiber quality sensor (S) as claimed in any of the preceding claims, wherein the first and/or second pairs of electrodes are tilted, slanted and/or zigzag-shaped.

5. A fiber quality sensor (S) as claimed in any of the preceding claims, for the or each pair of electrodes further comprising an amplifier coupled to the pair of electrodes and having an input impedance exceeding 1 TΩ, and preferably being 200 TΩ.

6. A hair care device, comprising:
a fiber quality sensor (S) as claimed in any of the preceding claims.
